# EUROPÄISCHE PATENTANMELDUNG

(11) **EP 3 446 718 A1**
(43) Veröffentlichungstag der Anmeldung: **27.02.2019**
(21) Anmeldenummer: 17187760.8
(22) Anmeldetag: 24.08.2017
(51) Int. Cl.: A61L 9/04, A61L 9/12

(54) **FORMKÖRPER ZUR VERWENDUNG ALS GERUCHSSTOFFRESERVOIR IN EINEM DUFTSTOFFSPENDER**

(71) Anmelder: Wittekind, Herbert, 7500 Silistra (BG)
(72) Erfinder: Wittekind, Herbert, 7500 Silistra (BG)
(74) Vertreter: EGLI-EUROPEAN PATENT ATTORNEYS

(57) **Zusammenfassung**

Die vorliegende Erfindung betrifft einen porösen luftdurchlässigen Formkörper, der eine Vielzahl von Kunststoffpartikeln umfasst, wobei die Kunststoffpartikel Duftstoffe umfassen, dadurch gekennzeichnet, dass wenigstens ein Teil der Kunststoffpartikel durch Verschmelzen eines Teils der Oberfläche der Kunststoffpartikel miteinander verbunden ist. Die Kunststoffpartikel sind bevorzugt kugelförmig und bestehen bevorzugt aus Ethylen/Vinylacetat-Copolymer. Weitere Aspekte der vorliegenden Erfindung sind ein Verfahren zur Herstellung eines solchen Formkörpers, ein durch dieses Verfahren hergestellter Formkörper und die Verwendung desselben als Geruchsstoffreservoir in einem Duftstoffspender.

## Beschreibung

### Einleitung

Die vorliegende Erfindung betrifft einen porösen luftdurchlässigen Formkörper, der eine Vielzahl von Kunststoffpartikeln umfasst, wobei die Kunststoffpartikel Duftstoffe umfassen, dadurch gekennzeichnet, dass wenigstens ein Teil der Kunststoffpartikel an ihren Berührungspunkten miteinander verschmolzen sind. Die Kunststoffpartikel sind bevorzugt kugelförmig und bestehen bevorzugt aus Ethylen/Vinylacetat-Copolymer. Weitere Aspekte der vorliegenden Erfindung sind ein Verfahren zur Herstellung eines solchen Formkörpers, ein durch dieses Verfahren hergestellter Formkörper und die Verwendung desselben als Geruchsstoffreservoir in einem Duftstoffspender.

### Stand der Technik

Aus verschiedensten Gründen kann der Bedarf bestehen, der Raumluft in geschlossenen Räumen Geruchsstoffe zuzusetzen, zum Beispiel, weil unangenehme Gerüche auftreten oder um das Wohlbefinden zu erhöhen. Eine grosse Anzahl von Methoden und Vorrichtungen wurden zu diesem Zweck geschaffen und kommerzialisiert. Sollen grosse Mengen von Geruchsstoffen freigesetzt werden, so werden die Geruchsstoffe in der Regel direkt verdampft. Bei geringeren Mengen wird häufig von Trägerstoffen Gebrauch gemacht, die mit Geruchsstoffen beladen und mit Luft in Kontakt gebracht werden.

Um einen Duft in die Umgebung abzugeben, sind verschiedene Vorrichtungen und Verfahren bekannt. Aus der EP 2 050 472 A1 ist zum Beispiel ein Duftstoffspender bekannt, der mit einem einen flüssigen Geruchsstoff enthaltenden Behälter und einem Ventilator ausgestattet ist. Solche Systeme sind in verschiedenen Abwandlungen gut bekannt. In solchen Systemen müssen jedoch Flüssigkeiten gehandhabt werden, was eine einfache Handhabung erschwert, komplizierte Apparate notwendig macht und zu Verschmutzungen führen kann. Solche Systeme sind daher für den Endverbraucher oft zu anspruchsvoll. Systeme, die mit Geruchsstoffen beladene partikuläre Trägerstoffe in einem Behälter aufweisen, sind zum Beispiel aus der WO 02/09779 A1 und der US 6,235,705 B1 bekannt. Sie sind sehr einfach zu handhaben und bedürfen lediglich des Ausbringens und Entfernen des Behälters. Ein Duftstoffspender oder irgendeine anderer Apparatur sind unnötig. Sie werden vor allem zum Eintragen von Geruchsstoffen in Waschmaschinen und Trockenmaschinen verwendet. Da der Behälter einen intensiven Stoffaustausch zwischen den mit Geruchsstoffen beladenen Partikeln und dem umgebenden Medium behindert, können nur geringe Mengen Geruchsstoff pro Zeiteinheit freigesetzt werden. Solche Systeme können daher nur eingesetzt werden, wenn der mit Geruchsstoffen zu versorgende Raum klein ist und/oder die Behälter intensiv von einem Medium umströmt werden. Für die Abgabe eines Geruchsstoffs in die Zimmerluft eines Raumes sind diese Systeme daher eher ungeeignet. Die Verwendung von Kunststoffen als Trägerstoffe für Geruchsstoffe ist zum Beispiel aus der WO 08/01503 A1 bekannt. Die Verwendung von Kunststoffpartikel zu diesem Zweck ist beispielsweise in der DE 102 37 066 A1 beschrieben. Solche Systeme, die Kunststoffe als Trägermaterialien verwenden, leiden jedoch darunter, dass die Geruchsstoffe allzu schnell abgegeben werden, was zunächst zu einer zu hohen Geruchsstoffkonzentration in der Luft und nach kurzer Zeit zu einer schnellen Erschöpfung des Geruchsstoffvorrats führt.

Für den Verbraucher ist es dabei wünschenswert, eine Möglichkeit zur Desodorierung von Luft zu erhalten, die möglichst einfach anwendbar ist, eine genügende Kapazität für die Desodorierung von Räumen aufweist, eine langanhaltende, gleichmässige Wirkung aufweist und preiswert ist.

### Aufgabe der Erfindung

Es war daher die Aufgabe der vorliegenden Erfindung, Produkte und Verfahren zu deren Herstellung zur Verfügung zu stellen, die es erlauben auf einfache Weise Luft zu desodorieren, wobei die Produkte so einfach wie möglich zu handhaben sein sollen, so dass auch Laien diese problemlos verwenden können. Die Produkte sollen ferner möglichst einfach herzustellen sein und sie sollen preiswert zu erwerben sein. Dabei soll die Kapazität zur Deodorierung möglichst gross sein. Weiter soll so weit möglich gewährleistet sein, dass der Effekt lange anhält. Ferner soll eine möglichst gleichmässige Abgabe des Geruchsstoffs an die Umgebung über eine lange Zeit erfolgen.

### Beschreibung der Erfindung

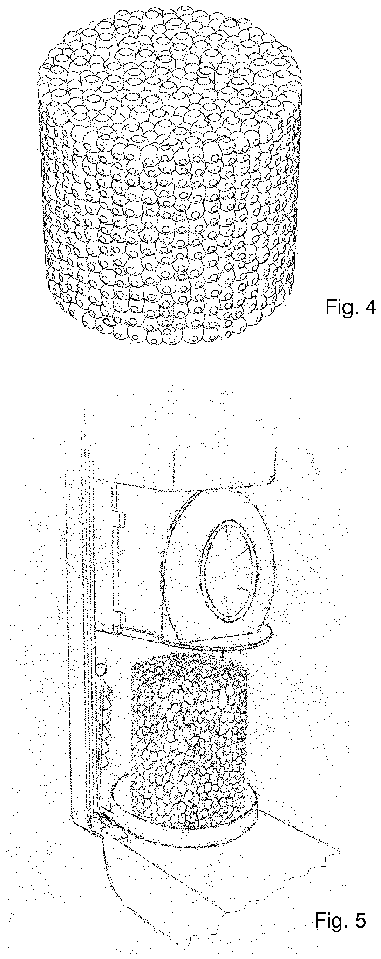

Die vorliegende Erfindung betrifft einen porösen luftdurchlässigen Formkörper, der eine Vielzahl von Kunststoffpartikeln umfasst, wobei die Kunststoffpartikel Duftstoffe umfassen, dadurch gekennzeichnet, dass wenigstens ein Teil der Kunststoffpartikel durch Verschmelzen eines Teils der Oberfläche der Kunststoffpartikel miteinander verbunden ist. Der erfindungsgemässe poröse luftdurchlässige Formkörper wird im Folgenden auch nur "erfindungsgemässer Formkörper" oder "Formkörper" genannt. Die duftbeladenen EVA Teile sind meist rundlich, und bei dem Übergang vom Erweichungspunkt zum Schmelzpunkt verkleben die EVA kugelähnlichen Teile nur an den Berührungspunkten und ermöglichen somit eine optimale Durchströmung des Gases durch den Duftkörper. Ein solcher erfindungsgemässer Formkörper ist in der Lage, die in den Kunststoffpartikeln enthaltenen Geruchsstoffe in die umgebende Raumluft zu entlassen. Im Gegensatz zur Verwendung von Kunststoffpartikeln in Form von Schüttgut, erlaubt diese Form des mit Geruchsstoff beladenen Kunststoffs eine einfache Handhabung des Kunststoffs, ohne dass die Notwenigkeit der Unterbringung in einem Behälter oder einer stützenden Struktur besteht. Dies vereinfacht und verbilligt die Herstellung und die Handhabung. Auch müssen keine Flüssigkeiten umständlich gehandhabt werden, die Verunreinigungen verursachen können. Dem erfindungsgemässen Formkörper kann eine fast beliebige Form verliehen werden, wie beispielsweise Würfelform, Quaderform, Kugelform, Zylinderform oder auch unregelmässige Formen. Die Form kann auch einem zu verwendenden Duftstoffspender angepasst sein, zum Beispiel einer zur Aufnahme des Geruchsstoffes vorgesehenen Aufnahmekammer des Duftstoffspenders (siehe zum Beispiel Abbildung 5). Der Formkörper, der in Duftstoffspendern als Geruchsstoffreservoir verwendet werden kann, kann dabei in einfacher Weise in eine solche Aufnahmekammer eingestellt werden. Da die erfindungsgemässen Formkörper so geformt sein können, dass sie auf einer ebenen Fläche stehen können, sind weder Stützhilfen noch Behälter oder dergleichen notwendig um diese zu installieren. Ist der Geruchsstoffvorrat in dem erfindungsgemässen Formkörper aufgebraucht, so kann der Formkörper auf die gleiche Art und Weise entnommen werden, wobei keine Verschmutzung oder Verunreinigung durch Flüssigkeiten auftreten und keine aufwändigen Apparaturen oder Anschlussverfahren verwendet werden müssen. Die erfindungsgemässen Formkörper können einen Raum je nach Grösse und Geruchsintensität, je nach Beladung des Formkörpers über einen Zeitraum von 4 bis 20 Wochen mit Geruchsstoffen versorgen.

Als Besonderheit weisen die erfindungsgemässen Formkörper auch ein anderes Abgabeprofil für den Geruchsstoff auf als nicht durch Verschmelzen eines Teils Oberfläche miteinander verbundene Kunststoffpartikel auf. Der Zweck der vorliegenden Formkörper ist es, die darin enthaltenen Geruchsstoffe freizusetzen und in ein umgebendes Medium einzubringen, wobei das Medium in der Regel Luft ist. Die erfindungsgemässen Formkörper können Ihre Wirkung daher am besten entfalten, wenn sie direkt dem stehenden oder strömenden Medium ausgesetzt werden. Dabei kann das Medium den Formkörper umfliessen und in ihn eindringen/durchdringen und mit der ganzen Oberfläche, einschliesslich der inneren Oberfläche der Formkörper wechselwirken und so ausreichende Mengen des Duftstoffs pro Zeiteinheit freisetzen.

Marktübliche, mit Geruchsstoffen beladene Kunststoffe haben jedoch den Nachteil, dass die Geruchsstoffabgabe stark zeitabhängig ist. Am Anfang werden grosse Mengen an Geruchsstoff pro Zeiteinheit an das Medium abgegeben, weil der Geruchsstoff, der sich in Oberflächennähe befindet, nur sehr kurze Distanzen durch den Kunststoff diffundieren muss, bis er verdampfen kann. Ist der Geruchsstoff nahe der Oberfläche der Kunststoffpartikel jedoch weitgehend verbraucht, so muss der Geruchsstoff aus tiefer liegenden Schichten an die Oberfläche diffundieren. Dadurch sinkt die Geruchsstoffabgabe mit der Zeit. Dies führt zu einem Abgabeprofil, bei dem am Anfang eine sehr grosse Menge an Geruchsstoff pro Zeiteinheit freigesetzt wird, die freigesetzte Menge aber schnell abnimmt und nach einer relativ kurzen Zeit nur noch geringe Mengen an Duftstoff pro Zeiteinheit abgegeben werden. Daher wird Anfangs zu viel Geruchsstoff an das Medium abgegeben und der Geruchsstoff ist nach kurzer Zeit verbraucht.

Die erfindungsgemässen Formkörper setzen am Anfang weniger Geruchsstoff pro Zeiteinheit frei, der Geruchsstoff wird jedoch dafür über einen längeren Zeitraum abgegeben. Man kann davon ausgehen, dass die bei der Herstellung der Formkörper entstehende Schmelzschicht an der Oberfläche der Kunststoffpartikel als Diffusionsbarriere wirkt, die eine schnelle Diffusion und Verdampfung des Geruchsstoffs verhindert und so eine retardierende und regulierende Wirkung ausübt. Dieser Effekt führt zu einer moderierten Geruchsstoffabgabe, die sehr vorteilhaft ist.

Luftdurchlässig im Sinne dieser Anmeldung bedeutet, dass Gase von einer Seite des Formkörpers durch den Formkörper auf eine andere Seite des Formkörpers fliessen können. Das Eindringen/Durchdringen des den Formkörper umgebenden Gases in den Formkörper wird so erleichtert und die Oberfläche des Kunststoffes erhöht, was eine optimale Duftaufnahme ermöglicht. Dies erhöht die Abgabegeschwindigkeit des Geruchsstoffes und erlaubt das Odorieren von grösseren Volumina. Der Formkörper wird bevorzugt in einen Gasstrom gestellt, was einen Gasstrom durch den Formkörper erzwingt und die Abgabegeschwindigkeit des Geruchsstoffes weiter erhöht und so, durch die Einstellung der Strömungsgeschwindigkeit, auf einfache Weise eine Steuerung der Abgabegeschwindigkeit des Geruchsstoffes erlaubt.

Die Herstellung des Formkörpers ist besonders einfach und kostengünstig, wenn er aus den Kunststoffpartikeln besteht, was daher bevorzugt ist.

Der erfindungsgemässe Formkörper weist bevorzugt eine Druckfestigkeit von wenigstens 0,1 N/mm² auf. Dies macht die Handhabung des Formkörpers einfach und verhindert Beschädigungen. Geringere Druckfestigkeiten könnten dazu führen, dass bei der Handhabung des Formkörpers durch Menschen oder Maschinen Beschädigungen daran auftreten und seine körperliche Integrität beeinträchtigt wird. Besonders bevorzugt weist der Formkörper eine Druckfestigkeit von wenigstens 0,5 N/mm², ganz besonders bevorzugt von wenigstens 1 N/mm² und am meisten bevorzugt von wenigstens 10 N/mm² auf.

Oft werden mit Geruchsstoffen beladene partikuläre Trägerstoffe von semipermeablen Barrieren umgeben, die die Festkörper zusammenhalten, aber den Durchtritt von Geruchstoffen in das umgebende Medium erlauben. Solche semipermeablen Barrieren sind oft Beutel aus Stoffen oder Behälter aus Karton oder Kunststoff, die Öffnungen enthalten, die kleiner sind als die Partikelgrösse. Solche semipermeablen Barrieren behindern freien Zutritt des umgebenden Mediums (in der Regel Luft) zu den erfindungsgemässen Formkörpern und damit zu den Geruchsstoffen und behindern damit auch den Übertritt der Geruchsstoffe in das Medium. Bevorzugt ist der erfindungsgemässe Formkörper daher nicht und zwar auch nicht teilweise von einer semipermeablen Barriere umgeben. Ganz besonders bevorzugt ist der erfindungsgemässe Formkörper überhaupt nicht von einer Barriere umgeben. Dabei wird unter Barriere jeder Gegenstand verstanden, der den freien Fluss des umgebenden Mediums in den Formkörper behindert. Nicht als Barriere in diesem Sinne gelten Standflächen oder Gegenstände, die der Installation der erfindungsgemässen Formkörper dienen.

Für Zwecke des Transports und der Lagerung ist jedoch eine Verpackung notwendig, die den Austritt des Geruchstoffes aus dem erfindungsgemässen Formkörper verhindert. Eine weitere Ausführungsform der vorliegenden Erfindung ist daher ein erfindungsgemässer Formkörper, der von einem Behälter umgeben ist, der für die Geruchsstoffe undurchlässig ist. Dabei kann es sich um eine Plastikfolie, eine Metalldose oder der gleichen handeln. Cellophan ist für diesen Zweck besonders geeignet. Bevorzugt sollte der Behälter so beschaffen sein, dass die Duftstoffleckage eine Rate von 400 ng/s besonders bevorzugt von 100 ng/s und ganz besonders bevorzugt von 10 ng/s nicht überschreitet. Dabei wird die Rate der Duftstoffleckage dadurch bestimmt, dass der verpackte Formkörper gewogen, im Behälter über eine bestimmte Zeit (zum Beispiel für einen Monat) bei 25°C und 1 bar an der Umgebungsluft gelagert und anschliessend noch einmal gewogen wird.

Die erfindungsgemässen Formkörper werden aus Kunststoffpartikel hergestellt, die mit Geruchsstoffen beladen sind. Solche Kunststoffpartikel sind dem Fachmann hinlänglich bekannt. Für die vorliegende Erfindung können alle bekannten Ausführungsformen solcher Kunststoffpartikel verwendet werden. Als Kunststoff kommen im Wesentlichen alle Kunststoffe in Frage, die sich mit Geruchsstoffen beladen lassen. Bevorzugt weist das Material der Kunststoffpartikel einen Schmelz- oder Erweichungspunkt im Bereich von 40 bis 125°C, vorzugsweise von 50 und 100°C, besonders bevorzugt von 60 bis 90°C und besonders bevorzugt von 70 bis 80°C auf.

Grundsätzlich sind alle Kunststoffe mit den vorstehend genannten Schmelz- oder Erweichungspunkten als Material für die Kunststoffpartikel geeignet. Bevorzugt sind erfindungsgemässe Formkörper, die dadurch gekennzeichnet sind, dass die Kunststoffpartikel eine oder mehrere Substanzen enthalten, die ausgewählt sind aus der Gruppe umfassend Ethylen/Vinylacetat-Copolymere, Polyethylen niederer oder hoher Dichte (LDPE, HDPE) oder Gemische derselben, Polypropylen, Polyethylen/Polypropylen-Copolymere, Polyether/Polyamid-Blockcopolymere, Styrol/Butadien-(Block-)Copolymere, Styrol/Isopren-(Block)Copolymere, Styrol/Ethylen/Butylen-Copolymere, Acrylnitril/Butadien/Styrol-Copolymere, Acrylnitril/Butadien-Copolymere, Polyetherester, Polyisobuten, Polyisopren, Ethylen/Ethylacrylat-Copolymere, Polyamide, Polycarbonat, Polyester, Polyacrylnitril, Polymethyl-methacrylat, Polyurethane und Polyvinylalkohole. Besonders bevorzugt umfassen die Kunststoffpartikel wenigstens ein Ethylen/Vinylacetat-Copolymer. Ethylen/Vinylacetat-Copolymere lassen sich bekanntermassen einfach mit grossen Mengen Geruchsstoffen beladen und die Geruchsstoffe enthaltenden Partikel lassen sich dann durch einfaches Erwärmen miteinander zu Formkörpern verschmelzen. Weitere geeignete Polymeren können den Absätzen [0011] bis [0043] der DE 102 37 066 A1 entnommen werden.

Besonders bevorzugt sind erfindungsgemässe Formkörper, die dadurch gekennzeichnet sind, dass die Kunststoffpartikel mindestens 10 Gew.-%, vorzugsweise mindestens 30 Gew.-%, besonders bevorzugt mindestens 70 Gew.-% Ethylen/Vinylacetat-Copolymer umfassen, jeweils bezogen auf die Gesamtmasse der in den Partikeln enthaltenen Polymeren und vorzugsweise als Polymere ausschliesslich Ethylen/Vinylacetat-Copolymer enthalten. Die Geruchsstoffe und etwaig andere enthaltene Substanzen werden bei diesen Angaben nicht berücksichtigt. Insoweit die Kunststoffpartikel wenigstens ein Ethylen/Vinylacetat-Copolymer enthalten, so ist es bevorzugt, das dieses wenigstens eine Ethylen/Vinylacetat-Copolymer 5 bis 50 Gew.-% Vinylacetat, vorzugsweise 7 bis 35 Gew.-% Vinylacetat und insbesondere 15 bis 30 Gew.-% Vinylacetat, jeweils bezogen auf das Gesamtgewicht des Ethylen/Vinylacetat-Copolymers, enthält. Das wenigstens eine Ethylen/Vinylacetat-Copolymer weist im Übrigen bevorzugt einen Schmelzindex von 0 bis 400, besonders bevorzugt von 0 bis 100 auf. Solche Polymere lassen sich einfach mit grossen Mengen Geruchsstoff beladen und die so erhaltenen Kunststoffpartikel lassen sich auf einfache Weise miteinander zu Formkörper verschmelzen. Die so entstehenden Formkörper haben eine ausreichende mechanische Festigkeit im Allgemeinen und eine hohe Druckfestigkeit im Besonderen.

Die Kunststoffpartikel der erfindungsgemässen Formkörper können beliebige Formen aufweisen, sie können zum Beispiel in Form von Kugeln, Zylindern, Würfeln, Quadern, Linsen, Tabletten und vielen anderen Formen verwendet werden. Handelsübliche Kunststoffpartikel haben in der Regel Tabletten-, Zylinder- der Kugelform. Bevorzugt sind Kunststoffpartikel, die eine Kugelform aufweisen. Der mittlere Durchmesser der in den erfindungsgemässen Formkörpern enthaltenen Kunststoffpartikel beträgt bevorzugt 0,1 bis 10 mm, besonders bevorzugt 0,5 bis 5 mm und ganz besonders bevorzugt von 1 bis 3 mm. Die Grösse wird bevorzugt durch Laserbeugung bestimmt.

Als Geruchsstoffe kommen alle bekannten Geruchsstoffe in Frage. Solche sind dem Fachmann bekannt. Geeignete Geruchsstoffe sind zum Beispiel beschrieben in "S. Arctander Perfume Flavors and Chemicals", Bände 1 und 2, Arctander, Montclair, NJ USA 1969. Als Geruchsstoffe können im Rahmen der vorliegenden Erfindung einzelne Geruchsstoffe, z.B. die synthetischen Produkte vom Typ der Ester, Ether, Aldehyde, Ketone, Alkohole und Kohlenwasserstoffe verwendet werden. Geruchsstoffe vom Typ der Ester sind z.B. Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, X-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Geruchsstoffe verwendet, die gemeinsam eine ansprechende Duftnote erzeugen. Solche Parfümöle können auch natürliche Geruchsstoffgemische enthalten, wie sie aus pflanzlichen Quellen zugänglich sind, z.B. Pine-, Citrus-, Jasmin-, Patchouly-, Rosen- oder Ylang-Yiang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Weitere geeignete Geruchsstoffe können dem Absatz [0051] der DE 102 37 066 A1 entnommen werden.

Der Gewichtsanteil der Geruchsstoffe in den erfindungsgemässen Formkörper beträgt bevorzugt 1 bis 70 Gew.-%, besonders bevorzugt 10 bis 60 Gew.-%, ganz besonders bevorzugt 20 bis 50 Gew.-% und am meisten bevorzugt 30 bis 40 Gew.-%, jeweils bezogen auf das Gesamtgewicht der Formkörper. Auch wenn es sich bei den Geruchsstoffen um Flüssigkeiten handelt, haben die erfindungsgemässen Formkörper bei solchen Beladungsraten immer noch die Eigenschaften von Festkörpern und lassen sich problemlos handhaben.

Ferner kann der erfindungsgemässe Formkörper weitere Substanzen enthalten, insbesondere solche die vom Fachmann üblicherweise in diesem Fachgebiet eingesetzt werden. Dabei handelt es sich bevorzugt um Substanzen, die ausgesucht sind aus der Gruppe der Parfümträger, Farbstoffe, antimikrobiellen Wirkstoffe, Herbizide, Fungizide, Antioxidantien oder Korrosionsinhibitoren. Diese Substanzen sind dabei bevorzugt Bestandteil der Kunststoffpartikel. Sie können aber auch zusätzlich zu den Kunststoffpartikeln im erfindungsgemässen Formkörper enthalten sein.

Ein weiterer Aspekt der vorliegenden Erfindung ist ein Verfahren zur Herstellung der erfindungsgemässen Formkörper. Ein grosser Vorteil der vorliegenden Erfindung liegt darin, dass die Kunststoffpartikel in sehr einfacher Weise durch Verschmelzen zu einem Formkörper verbunden werden können. Das erfindungsgemässe Verfahren umfasst die folgenden Schritte:
- Bereitstellen eines Schüttguts bestehend aus von mit Geruchsstoffen beladenen Kunststoffpartikeln,
- Erwärmen der Oberfläche bis zum Erreichen des Punktes zwischen Erweichen und Schmelzen. Die Schmelzpunkttemperatur darf hierbei nicht überschritten werden.
- Abkühlen der Kunststoffpartikel unter Bildung eines Formkörpers.

Das erfindungsgemässe Verfahren kann sehr einfach und schnell mit preiswerten Mitteln hergestellt werden und führt zu erfindungsgemässen Formkörpern mit den vorstehend beschriebenen Eigenschaften und Vorteilen. Das Beladen der Kunststoffpartikel mit dem Geruchsstoff kann dabei in einfacher Weise bei einer Temperatur von 15 bis 50°C, vorzugsweise von 20 bis 40°C, erfolgen. Hierzu werden die Partikel mit der entsprechenden Menge des Duftstoffs versetzt und durchmischt. Die Temperatur liegt aber bevorzugt unterhalb der Schmelz- oder Zersetzungstemperatur des Kunststoffs und auch unterhalb des Flammpunkts des Parfumöls, wobei die Kunststoffpartikel geringfügig quellen können.

Um die Formkörper mit bestimmten Formen zu versehen, werden die Kunststoffpartikel vor dem Verschmelzen in eine Form eingefüllt. Die Form kann offen oder teilweise oder ganz geschlossen sein. Bevorzugt ist daher ein Verfahren zur Herstellung eines erfindungsgemässen Formkörpers, umfassend die folgenden Schritte:
- Bereitstellen eines Schüttguts bestehend aus von mit Geruchsstoffen beladenen Kunststoffpartikeln,
- Füllen des Schüttguts in eine Form, die offen oder teilweise oder ganz geschlossen sein kann.
- Erwärmen der Oberfläche der Kunststoffpartikel bis zum Erreichen des Punktes zwischen Erweichen und Schmelzen. Die Schmelzpunkttemperatur darf hierbei nicht überschritten werden,
- Abkühlen der Kunststoffpartikel unter Bildung eines Formkörpers und
- Entfernen des Formkörpers aus der Form.

Das Material der Form kann jedes Material sein, das zum Formenbau geeignet ist. Bevorzugt ist es ausgesucht aus der Gruppe bestehend aus Kunststoffen aller Art, Holz, behandelt oder unbehandelt, beschichtet oder nicht beschichtet, Glas oder Glasverbundstoffe und Metalle aller Art. Bevorzugt sind Metallformen mit einer Antihaft-Beschichtung und andere Materialien, welche kleberesistente Eigenschaften aufweisen, wie Polypropylen.

Bevorzugt ist dabei ein erfindungsgemässes Verfahren, dadurch gekennzeichnet, dass das Erwärmen durch eine Methode erfolgt, die ausgewählt ist aus der Gruppe bestehend aus Erwärmen durch Mikrowellen, Erwärmen in einem Ofen, Erwärmen in einem Wärmetunnel und Erwärmen in einem heissen Gasstrom.

Die Temperatur, auf die die Partikel erwärmt werden, hängt dabei von verschiedenen Einflussfaktoren ab, wie zum Beispiel von dem Schmelz- oder Erweichungspunkt der mit den Geruchsstoffen beladenen Kunststoffpartikeln, von der Methode die zur Erhitzung verwendet wird und von der Menge der verwendeten Kunststoffpartikel. Im Allgemeinen liegt die Erhitzungstemperatur bei 25 bis 150°C. Die Dauer der Erwärmung beträgt bevorzugt von 5 Sekunden bis 5 Stunden, besonders bevorzugt von 1 Minute bis 30 Minuten und am meisten bevorzugt von 1 bis 10 Minuten, abhängig vom Erwärumgsverfahren, wie z.B. Erwärmen in einem Ofen, heißen Gasstrom, Mikrowelle oder Infrarotheizung. In der einfachsten Form des erfindungsgemässen Verfahrens wird die Form mit den Kunststoffpartikeln bis zum Beginn des Schmelzens oder des Erweichens der Oberfläche der Kunststoffpartikel erwärmt. Der Beginn des Schmelzens oder des Erweichens kann einfach durch Augenschein festgestellt werden. Dann wird das Erwärmen abgebrochen und der entstandene Formkörper abgekühlt.

Um die Eigenschaften der so hergestellten erfindungsgemässen Formkörper genau einstellen zu können, können Vorversuche für bestimmte Kunststoffpartikel mit bestimmten Geruchsstoffen durchgeführt werden. Zum Beispiel wird zunächst der Schmelz- und Erweichungspunkt der mit den Geruchsstoffen beladenen Kunststoffpartikel gemessen. In dem Herstellungsverfahren der erfindungsgemässen Formkörper wird dann vorzugsweise für 5 Minuten die Temperatur auf dem Punkt zwischen Erweichen und Schmelzen gehalten, erwärmt und wieder abgekühlt. Dann werden die gewünschten Eigenschaften gemessen und bei Abweichung von den gewünschten Eigenschaften werden die Temperatur und Zeitdauer der Erwärmung angepasst. In der Regel muss eine genügende Druckfestigkeit erreicht werden. Im Übrigen soll die thermische Belastung so gering wie möglich sein, damit nicht ein zu grosser Anteil der Geruchsstoffe verdampft und die Kunststoffpartikel nicht so weit verschmelzen, dass die Partikelzwischenräume verschlossen werden. Die notwendige Erhitzungsdauer und Temperatur können daher auch so ermittelt werden, dass zunächst, wie vorstehend beschrieben, der Schmelz- oder Erweichungspunkt der mit den Geruchsstoffen beladenen Kunststoffpartikel gemessen wird. In dem Herstellungsverfahren der erfindungsgemässen Formkörper wird dann für 5 Minuten bis zum Schmelz- oder Erweichungspunkt erwärmt und wieder abgekühlt. Anschliessend wird die Druckfestigkeit des entstandenen Formkörpers gemessen. Liegt die Druckfestigkeit im gewünschten Bereich, also zum Beispiel zwischen 0,5 und 1 N/mm², so sind Temperatur und Zeitdauer angemessen eingestellt. Liegt sie darüber, so wird die Temperatur erniedrigt und/oder die Zeitdauer verkürzt. Liegt sie darunter, so wird die Temperatur erhöht und/oder die Zeitdauer verlängert. Auf die gleiche Weise kann das Abgabeprofil der Geruchsstoffe für eine bestimmte Art von Formkörpern eingestellt werden. Zur Verringerung der anfänglichen Abgabemenge des Geruchsstoffs durch den Formkörper wird die Temperatur erhöht und/oder die Zeitdauer der Erwärmung verlängert und umgekehrt. Auf diese Weise können für alle möglichen mit den Geruchsstoffen beladenen Kunststoffpartikel geeignete Temperatur und Zeitdauer für den Erwärmungsvorgang ermittelt werden.

Der Gewichtsanteil der Geruchsstoffe in den bereitgestellten Kunststoffpartikeln beträgt bevorzugt 1 bis 70 Gew.-%, besonders bevorzugt 10 bis 60 Gew.-%, ganz besonders bevorzugt 20 bis 50 Gew.-% und am meisten bevorzugt 30 bis 40 Gew.-%, jeweils bezogen auf das Gesamtgewicht der Kunststoffpartikel. Auch wenn es sich bei den Geruchsstoffen um Flüssigkeiten handelt, haben die Kunststoffpartikel bei solchen Beladungsraten immer noch die Eigenschaften von Festkörpern und lassen sich problemlos zu Formkörpern verschmelzen.

Ein weiterer Gegenstand der vorliegenden Erfindung ist ein erfindungsgemässer Formkörper, wie hierin beschrieben, hergestellt nach einen erfindungsgemässen Verfahren.

Schliesslich ist die Verwendung eines erfindungsgemässen Formkörpers als Geruchsstoffreservoir in einem Duftstoffspender ebenfalls ein Gegenstand der vorliegenden Erfindung. Bevorzugt ist dabei die erfindungsgemässe Verwendung in einem Duftstoffspender, der einen Ventilator, und eine Aufnahmekammer umfasst, wobei die Aufnahmekammer den erfindungsgemässen Formkörper enthält und wobei der Ventilator Luft durch die Aufnahmekammer strömen lässt und so Geruchsstoff an die Luft abgegeben wird.

### Kurze Beschreibung der Abbildungen

- Abbildung 1:: Abbildung 1 zeigt mit Geruchsstoff beladene Kunststoffpartikel.
- Abbildung 2:: Abbildung 2 zeigt die Kunststoffpartikel von Abbildung 1 in einer würfelförmigen Form.
- Abbildung 3:: Abbildung 3 zeigt den aus dem Zusammenschmelzen der Kunststoffpartikel aus Abbildung 2 entstandenen erfindungsgemässen Formkörper.
- Abbildung 4:: Abbildung 4 zeigt einen weiteren erfindungsgemässen Formkörper.
- Abbildung 5:: Abbildung 5 zeigt den Formkörper gemäss Abbildung 4 in der geöffneten Aufnahmekammer eines Duftstoffspenders.

### Beispiele

### Beispiel 1:

100 g eines Ethylen-Vinylacetat-Copolymer mit einem Gehalt von 28 % Vinylacetat und einem Schmelzindex von 35, erhältlich von den Leuna-Werken T28035 unter dem Namen FLEXAREN® mit folgenden Produkteigenschaften als Richtwerte:

| **Eigenschaft** | **Richtwert** | **Einheit** | **Prüfmethode** |
|---|---|---|---|
| Schmelzindex (@ 190°C; 2,16kg) | 25 | g/10min | ISO 1133 |
| Dichte (@20°C) | 0,950 | g/cm³ | DIN EN ISO 1183-1 |
| VA-Gehalt | 28 | wt. -% | FTIR |
| Schmelztemperatur | 71 | °C | DSC |

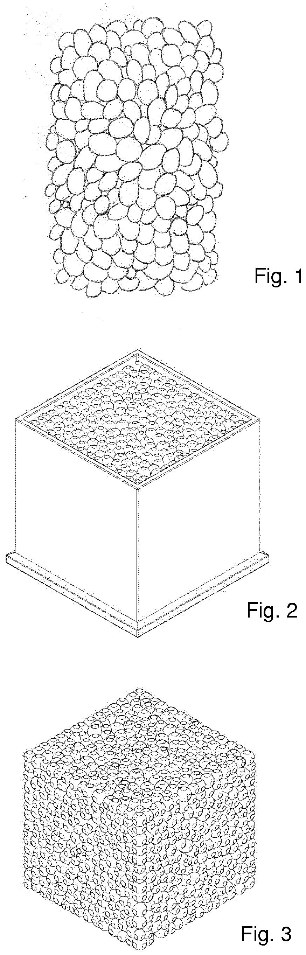

Die Partikel wurden mit 30 g Duftöl beladen (siehe Abbildung 1). Eine würfelförmige Kunststoffform mit den Innenabmessungen von 50 mm x 50 mm x 50 mm wird mit einem Teil dieser Kunststoffpartikel gefüllt (siehe Abbildung 2). Die Kunststoffform wird in einem Ofen auf 70°C während 45-120 Minuten erwärmt und anschliessend der Form entnommen (siehe Abbildung 3).

### Beispiel 2:

Entsprechend der Beschreibung von Beispiel 1 wurde ein zylinderförmiger Formkörper mit den Abmessungen 58 mm Durchmesser und 80 mm hoch hergestellt (siehe Abbildung 4) . Abbildung 5 zeigt diesen Formkörper in der Aufnahmekammer eines Duftstoffspenders.

## Patentansprüche

**1.** Poröser luftdurchlässiger Formkörper, der eine Vielzahl von Kunststoffpartikeln umfasst, wobei die Kunststoffpartikel Duftstoffe umfassen, **dadurch gekennzeichnet, dass** wenigstens ein Teil der Kunststoffpartikel durch Verschmelzen eines Teils der Oberfläche der Kunststoffpartikel miteinander verbunden ist.

**2.** Formkörper gemäss Anspruch 1, **dadurch gekennzeichnet, dass** er aus den Kunststoffpartikeln besteht.

**3.** Formkörper gemäss einem der Ansprüche 1 oder 2, **dadurch gekennzeichnet, dass** er eine Druckfestigkeit von wenigstens 0,1 N/mm² aufweist.

**4.** Formkörper gemäss einem der Ansprüche 1, 2 oder 3, **dadurch gekennzeichnet, dass** er nicht von einer semipermeablen Barriere umgeben ist.

**5.** Formkörper gemäss einem der Ansprüche 1 bis 4, insbesondere nach Anspruch 4, **dadurch gekennzeichnet, dass** er von einem Behälter umgeben ist, der für die Geruchsstoffe undurchlässig ist.

**6.** Formkörper gemäss einem der Ansprüche 1 bis 5, **dadurch gekennzeichnet, dass** die Kunststoffpartikel wenigstens ein Ethylen/Vinylacetat-Copolymer umfassen.

**7.** Formkörper gemäss einem der Ansprüche 1 bis 6, **dadurch gekennzeichnet, dass** die Kunststoffpartikel als Polymere ausschliesslich Ethylen/Vinylacetat-Copolymer enthalten.

**8.** Formkörper gemäss einem der Ansprüche 6 oder 7, **dadurch gekennzeichnet, dass** wenigstens ein in den Kunststoffpartikeln enthaltenes Ethylen/Vinylacetat-Copolymer 5 bis 50 Gew.-% Vinylacetat, vorzugsweise 7 bis 35 Gew.-% Vinylacetat und insbesondere 15 bis 30 Gew.-% Vinylacetat, jeweils bezogen auf das Gesamtgewicht des Ethylen/Vinylacetat-Copolymers, enthält.

**10.** Formkörper gemäss einem der Ansprüche 6 bis 8, insbesondere nach Anspruch 8, **dadurch gekennzeichnet, dass** das wenigstens ein in den Kunststoffpartikeln enthaltenes Ethylen/Vinylacetat-Copolymer einen Schmelzindex von 0 bis 400, bevorzugt von 0 bis 100 aufweist.

**11.** Formkörper gemäss einem der Ansprüche 1 bis 10, **dadurch gekennzeichnet, dass** der Gewichtsanteil der Duftstoffe 1 bis 70 Gew.-% bezogen auf das Gesamtgewicht des Formkörpers beträgt.

**12.** Formkörper gemäss einem der Ansprüche 1 bis 11, **dadurch gekennzeichnet**, das die Kunststoffpartikel einen mittleren Durchmesser von 0,1 bis 10 mm, gemessen nach der Laserbeugungsmethode, aufweisen.

**13.** Verfahren zur Herstellung eines Formkörpers gemäss einem der Ansprüche 1 bis 12, umfassend die folgenden Schritte:
- Bereitstellen eines Schüttguts bestehend aus von mit Geruchsstoffen beladenen Kunststoffpartikeln,
- Erwärmen der Oberfläche der Kunststoffpartikel bis zum Erreichen des Punktes zwischen Erweichen und Schmelzen
- Abkühlen der Kunststoffpartikel unter Bildung eines Formkörpers.

**14.** Verfahren nach Anspruch 13 umfassend die folgenden Schritte:
- Bereitstellen eines Schüttguts bestehend aus von mit Geruchsstoffen beladenen Kunststoffpartikeln,
- Füllen des Schüttguts in eine Form, die offen oder teilweise oder ganz geschlossen sein kann,
- Erwärmen der Oberfläche der Kunststoffpartikel bis zum Erreichen des Punktes zwischen Erweichen und Schmelzen des Kunststoffs,
- Abkühlen der Kunststoffpartikel unter Bildung eines Formkörpers und
- Entfernen des Formkörpers aus der Form.

**15.** Formkörper hergestellt nach einen Verfahren gemäss einem der Ansprüche 13 oder 14.

**16.** Verwendung eines Formkörpers gemäss einem der Ansprüche 1 bis 12 und 15, als Geruchsstoffreservoir in einem Duftstoffspender.

**17.** Verwendung gemäss Anspruch 20, wobei der Duftstoffspender einen Ventilator, und eine Aufnahmekammer umfasst, wobei die Aufnahmekammer einen Formkörper gemäss einem der Ansprüche 1 bis 12 und 15 enthält und wobei der Ventilator Luft durch die Aufnahmekammer strömen lässt und so Geruchsstoff vom Formkörper an die Luft abgegeben wird.
